# EUROPÄISCHE PATENTSCHRIFT

(11) **EP 3 266 366 B1**
(45) Veröffentlichungstag und Bekanntmachung des Hinweises auf die Patenterteilung: **06.05.2020**
(21) Anmeldenummer: 17179939.8
(22) Anmeldetag: 06.07.2017
(51) Int. Cl.: A61B 1/005, A61B 1/267

(54) **ADAPTIVES LARYNGOSKOP UND ADAPTIVER SPATEL FÜR EIN LARYNGOSKOP**
ADAPTIVE LARYNGOSCOPE AND ADAPTIVE BLADE FOR A LARYNGOSCOPE
LARYNGOSCOPE ADAPTATIF ET SPATULE ADAPTATIVE POUR UN LARYNGOSCOPE

(30) Priorität: 06.07.2016 DE 102016112385
(43) Veröffentlichungstag der Anmeldung: 10.01.2018
(73) Patentinhaber: Karl Storz SE & Co. KG, 78532 Tuttlingen (DE)
(72) Erfinder: Nettelroth, Vincent, 78259 Mühlhausen-Ehingen (DE); Hagen, Roland, 78549 Spaichingen (DE); Deppisch, Christopher, 78234 Engen (DE); Brechtold, Marcus, 78532 Tuttlingen (DE); Merz, Ulrich, 78532 Tuttlingen (DE); Fuhr, Eugenia, 78532 Tuttlingen (DE); Kupferschmid, Petra, 78532 Tuttlingen (DE); Efinger, Andreas, 78532 Tuttlingen (DE); Staud, Ralf, 78532 Tuttlingen (DE)
(74) Vertreter: Wimmer, Stephan

(56) Entgegenhaltungen:
- WO-A2-2016/074894
- CN-A- 106 236 001
- US-A1- 2013 310 650

## Beschreibung

Die vorliegende Erfindung ist auf ein adaptives Laryngoskop, insbesondere ein adaptives Intubationslaryngoskop oder ein adaptives Laryngoskop für die Larynxchirurgie oder andere Aufgaben innerhalb der Hals-Nasen-Ohren-Heilkunde bzw. Oto-Rhino-Laryngologie (engl.: Otorhinolaryngology), und auf einen adaptiven Spatel (engl.: blade) für ein solches Laryngoskop bezogen.

Zur endotrachealen Intubation in Anästhesie, Notfallmedizin und Intensivmedizin sowie der Chirurgie des Kehlkopfs (Larynx) werden für eine Intubation oder für chirurgische Maßnahmen ein unverlegter Zugang zum Kehlkopf, den Stimmbändern und letztlich der Trachea benötigt. Dabei dient ein Laryngoskop dazu, die Zunge nach vorne bzw. rostral zu schieben. Ein Laryngoskop umfasst in der Regel einen mehr oder weniger gekrümmten Spatel, an dessen proximalem Ende in näherungsweise rechtem Winkel ein Griff angeordnet ist.

Um eine Anpassung an die Anatomie des Patienten zu ermöglichen, ist der Spatel in der Regel austauschbar. In einem Intubationsset befindet sich eine Vielzahl von Spateln unterschiedlicher Länge und unterschiedlicher Krümmung. Für verschiedene Anwendungen und/oder unterschiedlicher Vorlieben des medizinischen Personals stehen ferner unterschiedliche Bauformen zur Verfügung, beispielsweise nach Macintosh, Miller, Dörges und McCoy, Letzterer mit einem bewegbaren distalen Ende.

Ein Laryngoskop mit einem verformbaren distalen Ende ist auch in WO 97/30626 beschrieben. Der Spatel 4 des Laryngoskops weist in einem mittleren Abschnitt 14 mehrere Schlitze 40 auf. Die Schlitze 40 unterteilen den mittleren Abschnitt 14 in Segmente 42, die nur durch schmale, als Festkörpergelenke wirkende Stege miteinander verbunden sind.

In EP 1 040 999 A2 ist ein Bauteil zur Aufnahme von Kräften beschrieben, bei dem Streben 11, 11a gegenüberliegende Bereiche einer Außenhaut 12, 12a miteinander verbinden. In EP 2 241 403 A1 ist ein Manipulatorwerkzeug mit zwei flexiblen Wangen 8, 10 beschrieben. Die Wangen 8, 10 sind am distalen Ende 6 des Manipulatorwerkzeugs 1 unmittelbar und im Übrigen durch mehrere Scharnierelemente 20 miteinander verbunden.

In DE 10 2007 026 721 A1 ist ein medizinisches Greifwerkzeug zum Halten von Körperbestandteilen beschrieben. Das medizinische Greifwerkzeug 1 umfasst mehrere Branchen 1 mit jeweils zwei gegenüberliegenden Wangen, zwischen denen verbindende Elemente verlaufen.

In WO 2016/074894 A2, die der Oberbegriff des Anspruchs 1 reflektiert, ist ein Intubationslaryngoskop mit einem Handriff 1 und einem gekrümmten Intubationsspatel 2 beschrieben. Der Intubationsspatel 2 umfasst einen biegbaren Spatelkörper aus zwei Spatelhalbschalen 2a, 2b und eine Spatelzunge 6, deren distales Ende im Bereich der Spatelspitze 3 fixiert ist. Die Spatelhalbschalen 2a, 2b bilden mit im rechten Winkel nach innen gebogenen Führungsschenkeln 11a, 11b eine Führung 11. Als T-Profile ausgebildete Führungsschlitten 13 an der Spatelzunge 6 greifen in die Führung 11 ein.

In US 2013/0310650 A1 ist ein Laryngoskop 1 mit einem Spatel 5 beschrieben. Eine Hülle 9, 80, 100 für den Spatel 5 kann an dem Laryngoskop 1 befestigt werden.

Eine Aufgabe der vorliegenden Erfindung besteht darin, einen verbesserten adaptiven Spatel für ein Laryngoskop und ein verbessertes adaptives Laryngoskop zu schaffen.

Diese Aufgabe wird durch die Gegenstände der unabhängigen Ansprüche gelöst.

Weiterbildungen sind in den abhängigen Ansprüchen angegeben.

Ein adaptiver Spatel für ein Laryngoskop umfasst ein proximales Ende, das mit einem Griffbauteil mechanisch verbindbar oder verbunden ist, um ein adaptives Laryngoskop zu bilden, einen ersten biegbaren Holm, der sich vom proximalen Ende des adaptiven Spatels bis zu dessen distalem Ende erstreckt, und einen zweiten biegbaren Holm, der sich vom proximalen Ende des adaptiven Spatels bis zu dessen distalem Ende erstreckt, wobei am distalen Ende des adaptiven Spatels die biegbaren Holme miteinander mechanisch starr oder gelenkig verbunden sind, und wobei die biegbaren Holme proximal des distalen Endes des adaptiven Spatels derart miteinander mechanisch verbunden sind, dass sie relativ zueinander im Wesentlichen in ihren Längsrichtungen bewegbar sind.

Der adaptive Spatel ist insbesondere zur Bildung eines Laryngoskops vorgesehen und ausgebildet, das zur Intubation oder für die Mikrolarynxchirurgie oder für andere Aufgaben innerhalb der Oto-Rhino-Laryngologie verwendbar ist. Das proximale Ende des adaptiven Spatels kann mit einem Griffbauteil dauerhaft - insbesondere für die gesamte vorgesehene Lebensdauer des Laryngoskops - und nicht zerstörungsfrei trennbar mechanisch verbunden sein. Insbesondere kann der adaptive Spatel teilweise oder ganz mit dem Griffbauteil einstückig ausgebildet sein, beispielsweise als gleichzeitig gefertigtes Gussteil aus Kunststoff, Metall oder einem anderen hinreichend elastischen Material. Alternativ kann am proximalen Ende des adaptiven Spatels eine Kupplungseinrichtung (beispielsweise in der Art einer Bajonett- bzw. Renkverbindung, einer Schraubverbindung, einer Rastverbindung) zur einmaligen oder wiederholbaren und zerstörungsfrei lösbaren Kopplung mit einem Griffbauteil vorgesehen sein.

Die beiden biegbaren Holme sind jeweils insbesondere bei den bei der vorgesehenen Verwendung auftretenden Kräften in Längsrichtung nicht oder nur geringfügig elastisch oder plastisch verformbar. Die elastische oder plastische Verformbarkeit der biegbaren Holme in Längsrichtung ist insbesondere so gering, dass eine Krümmung des adaptiven Spatels, die durch eine relative Verschiebung proximaler Enden der biegbaren Holme oder durch auf den adaptiven Spatel von außen einwirkende Kräfte bewirkt wird, im Vergleich zu idealisiert in Längsrichtung völlig unelastischen Holmen um nicht mehr als 50 % oder um nicht mehr als 20 % oder um nicht mehr als 10 % oder um nicht mehr als 5 % oder um nicht mehr als 2 % oder um nicht mehr als 1 % gemindert wird.

Die biegbaren Holme sind jeweils insbesondere nur in einer Richtung nennenswert elastisch und/oder plastisch biegbar und sind in einer dazu orthogonalen Richtung jeweils weitgehend biegesteif. Eine Biegbarkeit in einer ersten Richtung bzw. einer ersten Ebene und eine Biegesteifheit in einer dazu orthogonalen zweiten Richtung bzw. zweiten Ebene und eine geringe Verformbarkeit in Längsrichtung ist beispielsweise durch einen flachen Querschnitt eines Holms erzielbar, der in der ersten Richtung eine wesentliche kleinere Ausdehnung hat als in der zweiten Richtung. Beide biegbaren Holme sind jeweils insbesondere aus einem Polymer, einem anderen Kunststoff oder einem Metall gebildet.

Der erste biegbare Holm ist insbesondere vorgesehen und angeordnet, um bei der bestimmungsgemäßen Verwendung an der Zunge eines Patienten anzuliegen. Dazu ist der erste biegbare Holm insbesondere breit, flach und mit einer glatten zur Anlage an der Zunge vorgesehenen Oberfläche ausgebildet. Anstelle eines einzigen zweiten biegbaren Holms können zwei oder mehr, insbesondere parallel oder im Wesentlichen parallel zueinander angeordnete biegbare zweite Holme vorgesehen sein. Ein Raumbereich zwischen zwei zweiten biegbaren Holmen kann zum Einführen eines Tubus, eines Endoskops und/oder eines anderen medizinischen Instruments vorgesehen sein. Dazu kann ein Führungskanal mit offenem oder geschlossenem Querschnitt vorgesehen sein. Dieser Führungskanal kann mit dem ersten biegbaren Holm oder mit dem zweiten biegbaren Holm oder mit einem oder mehreren von mehreren zweiten biegbaren Holmen starr verbunden, insbesondere einstückig ausgebildet sein. Ferner kann eine Lichtquelle, beispielsweise eine Leuchtdiode, oder ein Lichtleitkabel zum Übertragen von Beleuchtungslicht, eine Kamera oder ein Bildsensor mit abbildender optischer Einrichtung oder ein flexibles Endoskop mit einem der biegbaren Holme, insbesondere an oder nahe dessen distalem Ende, verbindbar oder dauerhaft verbunden sein. Eine Verbindung ist dauerhaft, wenn sie ausgelegt ist, um während eines mehrere medizinische Eingriffe umfassenden Zeitraums oder während der gesamten vorgesehenen Lebensdauer des Laryngoskops zu bestehen.

Ein adaptiver Spatel mit hier beschriebenen Merkmalen und Eigenschaften kann durch medizinisches Personal an die Anatomie eines Patienten angepasst werden. Alternativ oder zusätzlich kann ein adaptiver Spatel mit hier beschrieben Merkmalen und Eigenschaften bei der vorgesehenen Verwendung durch elastische und/oder plastische Verformung selbsttätig sich an die Anatomie eines Patienten teilweise oder vollständig anpassen, insbesondere an Krümmung, Form und/oder Größe der Zunge oder des Rachenraums des Patienten. Diese Anpassung an die Anatomie des Patienten wird insbesondere ermöglicht durch die Biegeelastizität der beiden Holme und durch die mechanische Verbindung der beiden biegeelastischen Holme derart, dass die beiden biegeelastischen Holme relativ zu einander im Wesentlichen in ihren Längsrichtungen bewegbar sind, ihre Abstände aber nicht wesentlich veränderbar sind. Dadurch wird ein lediglich lokales Zurückweichen des adaptiven Spatels ermöglicht. Beispielsweise kann der adaptive Spatel an einem konvexen Bereich der Oberfläche der Zunge eines Patienten zurückweichen, und trotzdem kann ein distaler Bereich des adaptiven Spatels an einem konkaven Bereich der Oberfläche der Zunge anliegen. Dabei kann der adaptive Spatel einen vergleichsweise gleichmäßigen Druck auf die Oberfläche der Zunge ausüben, und zwar sowohl in konkaven Bereichen als auch in konvexen Bereichen der Oberfläche der Zunge.

Deshalb kann ein solcher adaptiver Spatel für Patienten mit unterschiedlichen anatomischen Charakteristika verwendbar sein und damit die Anzahl der vorzuhaltenden Spatel deutlich verringern. Außerdem kann insbesondere in der Notfallmedizin Zeit gewonnen werden, indem nicht erst ein Spatel ausgewählt und mit dem Griffstück verbunden werden muss, sondern ein bereits mit einem Griffstück verbundener adaptiver Spatel bei seiner Verwendung an die Anatomie des Patienten angepasst wird und/oder sich anpasst.

Ein adaptiver Spatel, wie er hier beschrieben ist, umfasst insbesondere ferner eine Strebe, die proximal des distalen Endes des adaptiven Spatels den ersten biegbaren Holm und den zweiten biegbaren Holm miteinander mechanisch verbindet.

Die Enden der Strebe sind insbesondere durch Festkörpergelenke oder andere Gelenke mit den biegbaren Holmen verbunden. Alternativ kann die gesamte Strebe als Festkörpergelenk bzw. elastisch ausgebildet sein.

Ein adaptiver Spatel, wie er hier beschrieben ist, umfasst insbesondere ferner ein dünnes und biegbares Verbindungsbauteil, das abwechselnd mit dem ersten biegbaren Holm und mit dem zweiten biegbaren Holm verbunden ist.

Das dünne und biegbare Verbindungsbauteil ist insbesondere biegeschlaff. Das dünne und biegbare Verbindungsbauteil umfasst insbesondere einen Draht, eine Faser, ein Filament, einen Faden oder ein dünnes Seil. Das dünne und biegbare Verbindungsbauteil kann beispielsweise Polyester, HPPE (High Performance Polyethylene; auch bekannt unter dem Markennamen Dyneema) oder ein anderes Polyethylen aufweisen. Das dünne und biegbare Verbindungsbauteil kann sich über den gesamten adaptiven Spatel oder einen Teil des adaptiven Spatels erstrecken.

Wenn der adaptive Spatel mehrere zweite biegbare Holme aufweist, können ein dünnes und biegbares Verbindungsteil zur Verbindung aller zweiten biegbaren Holme mit dem ersten biegbaren Holm vorgesehen sein. Alternativ können mehrere dünne und biegbare Verbindungsteile vorgesehen sein, wobei beispielsweise jedes dünne und biegbare Verbindungsbauteil zur Verbindung eines zweiten biegbaren Holms mit dem ersten biegbaren Holm vorgesehen ist.

Das dünne und biegbare Verbindungsbauteil ist insbesondere derart abwechselnd mit dem ersten biegbaren Holm und mit dem zweiten biegbaren Holm verbunden, dass es eine mäandernde Gestalt aufweist.

Diejenigen Abschnitte des dünnen und biegbaren Verbindungsbauteils, die die biegbaren Holme miteinander verbinden, sind insbesondere parallel oder im Wesentlichen parallel zueinander. Dies gilt insbesondere für jeweils benachbarte Abschnitte des dünnen und biegbaren Verbindungsbauteils.

Das dünne und biegbare Verbindungsbauteil kann Zugkräfte zwischen den biegbaren Holmen übertragen und einen maximalen Abstand der biegbaren Holme definieren.

Ein adaptiver Spatel, wie er hier beschrieben ist, umfasst insbesondere ferner Ösen oder andere Führungseinrichtungen an den biegbaren Holmen, wobei das dünne und biegbare Verbindungsbauteil abwechselnd durch Ösen oder andere Führungseinrichtungen am ersten biegbaren Holm und durch Ösen oder andere Führungseinrichtungen am zweiten biegbaren Holm geführt ist.

Jede Führungseinrichtung weist insbesondere eine ringförmige oder rohrförmige Gestalt auf. Insbesondere weisen die Ösen oder andere ringförmige Führungseinrichtungen an einem biegbaren Holm jeweils untereinander konstante oder im Wesentlichen konstante oder entlang des biegbaren Holms nur langsam variierende Abstände auf, und das dünne und biegbare Verbindungsbauteil ist jeweils abwechselnd durch zwei Führungseinrichtungen am ersten biegbaren Holm und durch zwei Führungseinrichtungen am zweiten biegbaren Holm geführt. Alternativ sind beispielsweise rohrförmige Führungseinrichtungen vorgesehen, wobei der Abstand zwischen zwei benachbarten Führungseinrichtungen jeweils im Wesentlichen der Länge einer Führungseinrichtung entspricht, wobei das dünne und biegbare Verbindungsbauteil jeweils abwechselnd durch eine Führungseinrichtung am ersten biegbaren Holm und durch eine Führungseinrichtung am zweiten biegbaren Holm geführt ist.

Bei einem adaptiven Spatel, wie er hier beschrieben ist, ist das dünne und biegbare Verbindungsbauteil in den Ösen oder anderen Führungseinrichtungen insbesondere jeweils in seiner Längsrichtung verschiebbar gelagert.

Dabei sind insbesondere die Enden des dünnen und biegbaren Verbindungsbauteils mit dem adaptiven Spatel, beispielsweise mit einem oder mehreren Enden der biegbaren Holme, fest verbunden. Eine Verschiebbarkeit des dünnen und biegbaren Verbindungsbauteils in den Ösen oder anderen Führungseinrichtungen kann einen weitgehenden Ausgleich mechanischer Spannungen ermöglichen und damit eine gleichmäßige Verformung des adaptiven Spatels unterstützen.

Alternativ kann das dünne und biegbare Verbindungsbauteil überall dort, wo es mit dem ersten biegbaren Holm oder mit dem zweiten biegbaren Holm verbunden ist, mit diesen starr oder zumindest nicht in seiner Längsrichtung verschiebbar verbunden sein.

Bei einem adaptiven Spatel, wie er hier beschrieben ist, umfasst das dünne und biegbare Verbindungsbauteil insbesondere einen Draht aus einer pseudoelastischen Nickel-Titan-Legierung oder einem anderen pseudoelastischen Material oder einen Draht aus einem anderen Material.

Ein Draht aus einer pseudoelastischen Nickel-Titan-Legierung (beispielsweise unter der Bezeichnung Nitinol bekannt) oder einem anderen pseudoelastischen und/oder elastischen Material (beispielsweise Metall, Polyester oder einem anderen Kunststoff) kann anders als ein Faden oder ein Seil eine geschlossene und porenfreie Oberfläche aufweisen, die eine Reinigung und Sterilisation deutlich vereinfachen kann.

Ein adaptiver Spatel, wie er hier beschrieben ist, umfasst insbesondere ferner einen bogenförmigen Abschnitt am zweiten Ende des zweiten biegbaren Holms.

Der bogenförmige Abschnitt am zweiten Ende des zweiten biegbaren Holms ist insbesondere kreisbogenförmig oder im Wesentlichen kreisbogenförmig.

Ein adaptiver Spatel, wie er hier beschrieben ist, umfasst insbesondere ferner Speicheneinrichtungen, deren erste Enden mit dem proximalen Ende des ersten biegbaren Holms mechanisch verbunden sind, und deren zweite Enden mit dem bogenförmigen Abschnitt des zweiten biegbaren Holms mechanisch verbunden sind.

Die ersten bzw. radial inneren Enden der Speicheneinrichtungen sind insbesondere jeweils gelenkig - unmittelbar oder mittelbar - mit dem proximalen Ende des ersten biegbaren Holms mechanisch verbunden. Die zweiten bzw. radial äußeren Enden der Speicheneinrichtungen sind insbesondere jeweils gelenkig mit dem bogenförmigen Abschnitt mechanisch verbunden. Die gelenkigen Verbindungen können als Festkörpergelenke oder als auf Formschluss beruhende Gelenke ausgebildet sind. Die Speicheneinrichtungen können jeweils über ihre gesamte Länge dünn und biegbar ausgebildet sein, um eine Verformung und eine Materialermüdung nicht unnötig an einem Ort zu konzentrieren.

Der bogenförmige Abschnitt am proximalen Ende des zweiten biegbaren Holms, geführt durch die beschriebenen Speicheneinrichtungen oder auf andere Weise, kann eine Umlenkung von Kräften und Bewegungen ermöglichen. Insbesondere können Längsbewegungen des zweiten biegbaren Holms (und entsprechende Zug- oder Druckkräfte im zweiten biegbaren Holm) durch den bogenförmigen Abschnitt mit Bewegungen eines Bedienelements, das parallel zu einem mit dem Spatel verbundenen Griff bewegbar ist, (und entsprechenden auf das Bedienelement manuell ausgeübten Kräften) gekoppelt werden.

Ein adaptiver Spatel, wie er hier beschrieben ist, umfasst insbesondere ferner ein elastisches Mantelbauteil zum Schutz des adaptiven Spatels vor Verschmutzung und anderen Umwelteinflüssen.

Das elastische Mantelbauteil weist insbesondere eine elastische Folie, ein elastisches Gewebe, ein elastisches Gewirk und/oder ein elastisches Vlies oder ein anderes elastisches blatt-, folien-, blech- oder textilartiges Material auf. Das elastische Mantelbauteil kann mit dem adaptiven Spatel dauerhaft verbunden oder für einen Austausch nach jeder Verwendung vorgesehen sein. Eine Verbindung ist dauerhaft, wenn sie ausgelegt ist, um während eines mehrere medizinische Eingriffe umfassenden Zeitraums oder während der gesamten vorgesehenen Lebensdauer des Laryngoskops zu bestehen.

Ein adaptiver Spatel, wie er hier beschrieben ist, umfasst insbesondere ferner eine formschlüssige Verbindung der beiden biegbaren Holme proximal des distalen Endes des adaptiven Spatels, die eine Bewegung der beiden biegbaren Holme relativ zu einander in einer ersten vorbestimmten Richtung ermöglicht und eine Bewegung der beiden biegbaren Holme relativ zu einander in einer zweiten vorbestimmten Richtung, die zu der ersten vorbestimmten Richtung orthogonal ist, formschlüssig unterbindet.

Der adaptive Spatel kann mehrere formschlüssige Verbindungen der beiden biegbaren Holme aufweisen. Die erste vorbestimmte Richtung ist insbesondere parallel oder im Wesentlichen parallel zu mindestens einem der beiden biegbaren Holme bzw. zu dessen Längsrichtung. Die formschlüssige Verbindung unterbindet eine Bewegung der beiden biegbaren Holme relativ zueinander in einer dazu orthogonalen Richtung oder in allen dazu orthogonalen Richtungen insbesondere bis auf Spiel, das unvermeidbar ist oder aus fertigungstechnischen Gründen nicht vermieden wird.

Bei einem adaptiven Spatel, wie er hier beschrieben ist, ist die formschlüssige Verbindung insbesondere durch eine erste Einrichtung am ersten biegbaren Holm und eine zweite Einrichtung am zweiten biegbaren Holm gebildet, wobei ein konvexer Bereich an der ersten Einrichtung am ersten biegbaren Holm in einen konkaven Bereich an der zweiten Einrichtung am zweiten biegbaren Holm eingreift und innerhalb dessen in der ersten vorbestimmten Richtung verschiebbar ist.

Alternativ oder zusätzlich kann ein konvexer Bereich an der zweiten Einrichtung am zweiten biegbaren Holm in einen konkaven Bereich an der ersten Einrichtung am ersten biegbaren Holm eingreifen und innerhalb dessen in der ersten vorbestimmten Richtung verschiebbar sein.

Ein konvexer Bereich umfasst insbesondere eine Nase oder einen Steg. Ein korrespondierender konkaver Bereich umfasst insbesondere eine Nut oder einen Schlitz, in die bzw. den die Nase oder der Steg eingreift. Insbesondere umfasst die erste Einrichtung am ersten biegbaren Holm einen T- oder L-förmigen Bereich, der zum zweiten biegbaren Holm hin ragt, und die zweite Einrichtung am zweiten biegbaren Holm umfasst einen oder mehrere T- oder L-förmige Bereiche, die zum ersten biegbaren Holm hin ragen, wobei zu den biegbaren Holmen parallele oder im Wesentlichen parallele balkenförmige Abschnitte oder Abschnitte mit balkenförmigen Querschnitten der T- oder L-förmigen Bereiche einander hintergreifen.

Die formschlüssige Verbindung der beiden biegbaren Holme an einem Ort proximal des distalen Endes des adaptiven Spatels kann eine Verbindung der biegbaren Holme mit konstantem Abstand ermöglichen, die, anders als bei einer Strebe, die selbst oder deren Enden als Festkörpergelenke ausgebildet sind, keine elastischen Rückstellkräfte bewirkt.

Ein adaptiver Spatel, wie er hier beschrieben ist, umfasst insbesondere ferner einen Kanal, in den zumindest entweder ein Endoskop oder eine Lichtquelle oder ein anderes medizinisches Instrument eingeführt werden kann.

Der Kanal kann teilweise oder vollständig mit dem ersten biegbaren Holm oder mit dem zweiten biegbaren Holm verbunden sein. Insbesondere kann der Kanal teilweise oder vollständig mit dem ersten biegbaren Holm oder mit dem zweiten biegbaren Holm einstückig ausgebildet sein. Der Kanal kann teilweise, weitgehend oder vollständig einen geschlossenen Querschnitt aufweisen. Der Kanal kann teilweise, weitgehend oder vollständig einen offenen Querschnitt, insbesondere einen auf einer Seite nicht ganz geschlossenen Querschnitt aufweisen. Dies kann insbesondere dann die Herstellung vereinfachen, wenn der Kanal und zumindest einer der beiden biegbaren Holme als gemeinsames Gussteil hergestellt werden.

Der Kanal kann aus einer Mehrzahl oder einer Vielzahl von Segmenten, die jeweils ringförmig oder rohrförmig sein und/oder einen U-förmigen Querschnitt aufweisen können, bestehen, die durch schmale Spalte oder Abstände getrennt sein können. Dadurch kann eine Versteifung eines biegbaren Holm, mit dem der Kanal verbunden ist, durch den Kanal vermieden werden.

Ein adaptiver Spatel, wie er hier beschrieben ist, kann für eine mehrmalige Verwendung und mehrmalige Sterilisation (insb. Dampfsterilisation im Autoklaven) oder für eine einmalige Verwendung und nachfolgende Entsorgung vorgesehen und ausgebildet sein.

Ein adaptives Laryngoskop umfasst einen adaptiven Spatel, wie er hier beschrieben ist, und ein Griffbauteil, das mit dem proximalen Ende des adaptiven Spatels mechanisch verbindbar oder verbunden ist.

Das adaptive Laryngoskop ist insbesondere ein Intubationslaryngoskop und/oder für eine Verwendung in der Mikrolarynxchirurgie oder für andere Anwendungen in der Oto-Rhino-Laryngologie vorgesehen.

Der adaptive Spatel kann teilweise oder vollständig mit dem Griffbauteil einstückig ausgebildet sein, beispielsweise als gleichzeitig hergestelltes Gussteil.

Ein adaptives Laryngoskop, wie es hier beschrieben ist, umfasst insbesondere ferner eine Einrichtung zum manuellen Bewegen des proximalen Endes des zweiten biegbaren Holms relativ zum proximalen Ende des ersten biegbaren Holms.

Dabei können mittels der Einrichtung das proximale Ende des zweiten biegbaren Holms und/oder das proximale Ende des ersten biegbaren Holms relativ zum Griffbauteil bewegbar sein. Die Einrichtung zum manuellen Bewegen umfasst beispielsweise einen Hebel. Der Hebel kann um eine Schwenkachse, die orthogonal zum adaptiven Spatel und orthogonal zum Griffbauteil ist, schwenkbar sein. Alternativ kann die Einrichtung zum manuellen Bewegen eine Drucktaste oder einen Schieber, beispielsweise einen parallel zum Griffbauteil verschiebbaren Schieber, aufweisen.

Ein adaptives Laryngoskop, wie es hier beschrieben ist, umfasst insbesondere ferner eine Einrichtung zum alternativen Arretieren des proximalen Endes von einem der beiden biegbaren Holme in einer von mehreren alternativen Positionen relativ zum proximalen Ende des anderen der beiden biegbaren Holme.

Die Einrichtung kann zum alternativen Arretieren in einer von mehreren alternativen vorbestimmten Positionen (beispielsweise durch Rastung oder auf andere Weise formschlüssig) oder zum Arretieren in beliebigen Positionen (beispielsweise durch Klemmung) ausgebildet sein.

Ein adaptives Laryngoskop, wie es hier beschrieben ist, umfasst insbesondere ferner eine Skala am Griffbauteil, an der Einrichtung zum manuellen Bewegen des proximalen Endes des zweiten biegbaren Holms relativ zum proximalen Ende des ersten biegbaren Holms, an der Einrichtung zum alternativen Arretieren, am adaptiven Spatel oder an anderer Stelle, die eine Identifizierung oder Zuordnung oder Reproduktion verschiedener relativer Positionen der proximalen Enden der biegbaren Holme vereinfacht.

Bei einem Verfahren zum Anpassen eines Laryngoskops an einen Patienten werden proximale Enden von biegbaren Holmen eines Spatels des Laryngoskops relativ zueinander verschoben.

Das Verfahren ist insbesondere auf adaptive Laryngoskope, wie sie hier beschrieben sind, und auf Laryngoskope mit adaptiven Spateln, wie sie hier beschrieben sind, anwendbar. Das Verschieben der proximalen Enden der biegbaren Holme des Spatels des Laryngoskops erfolgt insbesondere manuell mittels eines Hebels, eines Schiebers oder einer Drucktaste.

Bei einem Verfahren zum Anpassen eines Laryngoskops, wie es hier beschrieben ist, werden insbesondere die proximalen Enden der biegbaren Holme in einer von mehreren vorbestimmten relativen Positionen oder in einer beliebigen relativen Position arretiert.

### Kurzbeschreibung der Figuren

Nachfolgend werden Ausführungsformen anhand der beigefügten Figuren näher erläutert. Es zeigen:
- Figur 1: eine schematische axonometrische Darstellung eines adaptiven Laryngoskops;
- Figur 2: eine weitere schematische axonometrische Darstellung des adaptiven Laryngoskops aus Figur 1;
- Figur 3: eine schematische axonometrische Darstellung eines weiteren adaptiven Laryngoskops;
- Figur 4: eine schematische Darstellung eines weiteren adaptiven Laryngoskops;
- Figur 5: eine schematische Darstellung eines weiteren adaptiven Laryngoskops;
- Figur 6: eine schematische Darstellung eines Querschnitts durch das adaptive Laryngoskop aus Figur 5;
- Figur 7: eine schematische Darstellung eines Querschnitts durch ein weiteres adaptives Laryngoskop;
- Figur 8: eine schematische Darstellung eines Querschnitts durch ein weiteres adaptives Laryngoskop.

### Beschreibung der Ausführungsformen

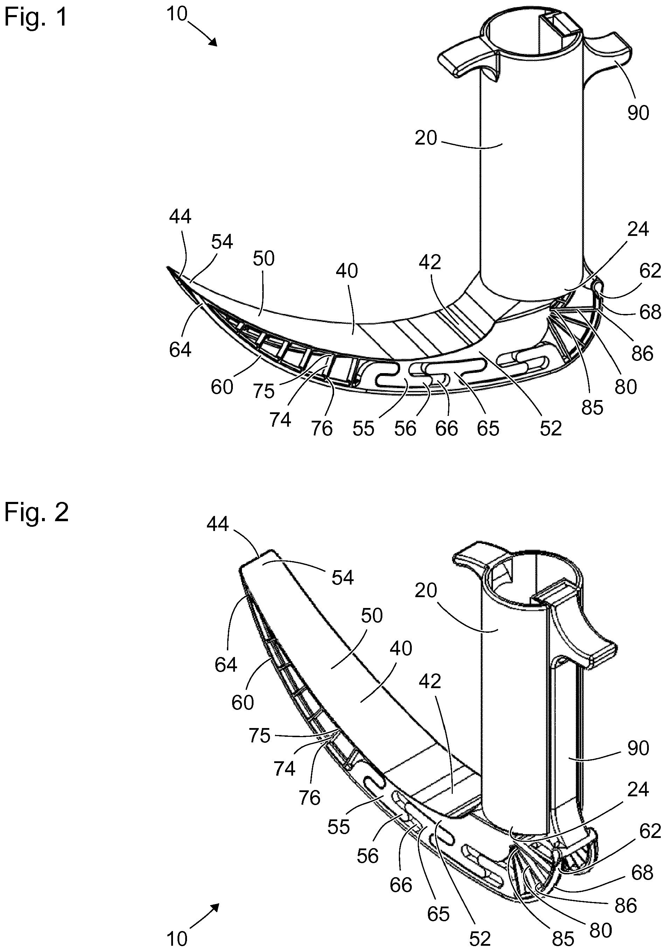

Figur 1 zeigt eine schematische axonometrische Darstellung eines adaptiven Laryngoskops, nämlich eines adaptiven Intubationslaryngoskops 10 mit einem Griffbauteil 20 zum manuellen Halten und Führen des Intubationslaryngoskops 10. Ferner umfasst das adaptive Intubationslaryngoskop 10 einen adaptiven Spatel 40 mit einem proximalen Ende 42 und einem distalen Ende 44. Der adaptive Spatel 40 ist leicht gekrümmt und weist einen zum distalen Ende 44 hin sich vermindernden Querschnitt auf. Das proximale Ende 42 des adaptiven Spatels 40 ist derart mit dem Griffbauteil 20 mechanisch verbunden, dass der an das proximale Ende angrenzende Bereich des adaptiven Spatels 40 mit dem Griffbauteil 20 einen Winkel von näherungsweise 90 Grad (im Bereich zwischen ca. 80 Grad und 120 Grad) einschließt.

Bei dem dargestellten Beispiel sind das Griffbauteil 20 und der adaptive Spatel 40 weitgehend einstückig ausgebildet, bestehen insbesondere weitgehend aus einem einzigen Kunststoff-Gussteil. Alternativ und abweichend von der Darstellung in Figur 1 kann im Übergangs- bzw. Verbindungsbereich 24 zwischen dem Griffbauteil 20 und dem adaptiven Spatel 40 eine Kupplungseinrichtung zur einmaligen oder wiederholten und zerstörungsfreien, lösbaren mechanischen Kopplung des Griffbauteils 20 und des adaptiven Spatels 40 vorgesehen sein.

Der adaptive Spatel 40 weist einen ersten biegbaren Holm 50 und zwei zweite biegbare Holme 60 auf. Bei der Darstellung in Figur 1 ist nur einer der beiden zweiten biegbaren Holme 60 vollständig sichtbar, der andere ist hinter dem ersten biegbaren Holm 50 verborgen.

Der erste biegbare Holm 50 weist im Wesentlichen einen breiten und flachen rechteckigen Querschnitt auf. Die in Figur 1 sichtbare und zur Anlage an einer Zunge eines Patienten vorgesehene Oberfläche des ersten biegbaren Holms 50 ist glatt oder weitgehend glatt. Die zweiten biegbaren Holme 60 weisen jeweils im Wesentlichen einen quadratischen Querschnitt auf. Der Abstand der zweiten biegbaren Holme 60 entspricht im Wesentlichen der Breite des ersten biegbaren Holms 50.

Die zweiten biegbaren Holme 60 sind nahe dem proximalen Ende 42 des adaptiven Spatels 40 im Wesentlichen parallel zu den Rändern des ersten biegbaren Holms 50. Zum distalen Ende 44 des adaptiven Spatels 40 hin nehmen die Abstände der zweiten biegbaren Holme 60 von den Rändern des ersten biegbaren Holms 50 ab. Auch die Breite des ersten biegbaren Holms 50 und entsprechend der Abstand der beiden zweiten biegbaren Holme voneinander nimmt zum distalen Ende 44 des adaptiven Spatels 40 hin etwas ab. Am distalen Ende 44 des adaptiven Spatels 40 sind der erste biegbare Holm 50 und die zweiten biegbaren Holme 60 miteinander mechanisch starr verbunden.

In einem Bereich, der sich näherungsweise vom proximalen Ende 42 bis zur Mitte des adaptiven Spatels 40 erstreckt, sind an den Rändern des ersten biegbaren Holms 50 T-förmige Einrichtungen 55 und an den zweiten biegbaren Holme 60 L-förmige oder T-förmige Einrichtungen 65 vorgesehen. Die T-förmigen Einrichtungen 55 am ersten biegbaren Holm 50 sind zu den zweiten biegbaren Holmen 60 hin orientiert und liegen an diesen an oder weisen nur einen geringen Abstand von diesen auf. L-förmige oder T-förmige Einrichtungen 65 an den zweiten biegbaren Holmen 60 sind zu den gegenüberliegenden Rändern des ersten biegbaren Holms 50 hin orientiert und liegen an diesen an oder weisen einen geringen Abstand von diesen auf.

Jeweils zwischen einem biegbaren Holm 50, 60 einerseits und dem Querbalken einer mit dem gleichen biegbaren Holm 50, 60 verbundenen der L- oder T-förmigen Einrichtung 55, 65 liegen schlitzartige Bereiche 66 vor. Die T-förmigen Einrichtungen 55 an den Rändern des ersten biegbaren Holms 50 und die L-förmigen oder T-förmigen Einrichtungen 65 an den zweiten biegbaren Holmen 60 hintergreifen einander. Dabei ist beispielsweise ein balkenförmiger Bereich 56 an der T-förmigen Einrichtung 55 am ersten biegbaren Holm 50 in einem schlitzartigen Bereich 66 zwischen einem der beiden zweiten biegbaren Holme 60 einerseits und der T-förmigen Einrichtung 65 daran anderseits angeordnet. Durch das gegenseitige Hintergreifen der L-förmigen und T-förmigen Einrichtungen 55, 65 sind die zweiten biegbaren Holme 60 relativ zum ersten biegbaren Holm 50 zwar innerhalb vorbestimmter Grenzen in ihrer Längsrichtung verschiebbar, die Abstände der zweiten biegbaren Holme 60 von den Rändern des ersten biegbaren Holms 50 sind jedoch formschlüssig definiert und können weder vergrößert noch verkleinert werden.

In einem distalen Bereich, der sich im Wesentlichen von der Mitte bis zum distalen Ende 44 des adaptiven Spatels 40 erstreckt, sind die zweiten biegbaren Holme 60 mit den jeweils gegenüberliegenden Rändern des ersten biegbaren Holms 50 durch Streben 74 verbunden. Die Streben 74 weisen Querschnitte auf, die in der Art eines Festkörpergelenks eine Verformung, bei den bei der vorgesehenen Verwendung auftretenden Kräften jedoch keine nennenswerte Änderung der Länge zulassen. Dadurch sind die Abstände der zweiten biegbaren Holme 60 von den jeweils gegenüberliegenden Rändern des ersten biegbaren Holms 50 im Wesentlichen unveränderbar festgelegt bzw. definiert, die zweiten biegbaren Holme 60 können jedoch relativ zu den gegenüberliegenden Rändern des ersten biegbaren Holms 50 verschoben werden. Dabei ist die Relativbewegung der zweiten biegbaren Holme 60 und des ersten biegbaren Holms 50 orthogonal oder im Wesentlichen orthogonal zu den Streben 74 und damit bei der in Figur 1 angedeuteten Konfiguration parallel oder im Wesentlichen parallel zu den biegbaren Holmen 50, 60.

An den proximalen Enden 62 der zweiten biegbaren Holme 60 sind bogenförmige, insbesondere im Wesentlichen kreisbogenförmige Abschnitte 68 vorgesehen. Zwischen den kreisbogenförmigen Abschnitten 68 und dem Griffbauteil 20, das an dieser Stelle in das proximale Ende 52 des ersten biegbaren Holms 50 übergeht, sind im Wesentlichen radial angeordnete Speicheneinrichtungen 80 vorgesehen. Erste, radial innere Enden 85 der Speicheneinrichtungen 80 sind mit dem Griffbauteil 20 und über dieses mittelbar mit dem proximalen Ende 52 des ersten biegbaren Holms 50 mechanisch verbunden. Radial äußere zweite Enden 86 der Speicheneinrichtungen 80 sind mit den bogenförmigen Abschnitten 68 verbunden. Die gesamten Speicheneinrichtungen 80 oder zumindest deren Enden 85, 86 sind biegbar bzw. als Festkörpergelenke ausgebildet. Die Querschnitte der Speicheneinrichtungen 80 sind so gewählt, dass sie bei den bei der vorgesehenen Verwendung des Intubationslaryngoskops 10 auftretenden Kräften nicht oder im Wesentlichen nicht ihre Länge verändern.

Am Griffbauteil 20 ist ein Schieber 90 vorgesehen, der relativ zum Griffbauteil 20 und parallel zu diesem manuell verschoben werden kann. Ein Ende des Schiebers 90 ist mit den bogenförmigen Abschnitten 68 bzw. den proximalen Enden der zweiten biegbaren Holme 60 mechanisch gekoppelt.

Die bogenförmigen Abschnitte 68 und die Speicheneinrichtungen 80 bewirken, dass eine Verschiebung des Schiebers 90 parallel zum Griffbauteil 20 mit einer Verschiebung der zweiten biegbaren Holme 60 relativ zum ersten biegbaren Holm 50 einhergeht. Die bogenförmigen Abschnitte 68 und die Speicheneinrichtungen 80 bewirken dabei eine Änderung der Richtung von Kraft und Weg um ca. 90 Grad.

Da am distalen Ende 44 des adaptiven Spatels 40 die biegbaren Holme 50, 60 miteinander mechanisch starr verbunden sind, bewirkt eine Verschiebung der zweiten biegbaren Holme 60 relativ zum ersten biegbaren Holm 50 eine Veränderung der Krümmung des adaptiven Spatels 40. Die Krümmung des adaptiven Spatels 40 kann also durch Verschieben des Schiebers 90 am Griffbauteil 20 verändert werden. Zur Fixierung einer eingestellten Krümmung des adaptiven Spatels 40 kann eine in Figur 1 nicht gezeigte Rasteinrichtung oder andere Feststelleinrichtung vorgesehen sein.

Figur 2 zeigt eine weitere schematische axonometrische Darstellung des anhand der Figur 1 dargestellten adaptiven Intubationslaryngoskops 10. Die Darstellung in Figur 2 unterscheidet sich von der in Figur 1 vor allem durch die andere Blickrichtung.

In Figur 2 ist erkennbar, dass der Schieber 90 sich entlang des gesamten Griffbauteils 20 erstreckt. Der Schieber 90 ist in einer Nut oder einem offenen Kanal formschlüssig geführt. An dem vom adaptiven Spatel 40 abgewandten Ende des Griffbauteils 20 weist der Schieber 90 eine Nase oder einen Vorsprung auf, der seine manuelle Verschiebung vereinfacht.

An dem dem adaptiven Spatel 40 zugewandten Ende des Griffbauteils 20 ist der Schieber 90 mit den proximalen Enden 62 der zweiten biegbaren Holme 60 mechanisch verbunden. Insbesondere ist der Schieber 90 mit den biegbaren Holmen 60 durch Festkörpergelenke oder andere Gelenke oder starr verbunden. Der Schieber 90 und die biegbaren Holme 60 können durch ein gemeinsam hergestelltes Gussteil aus Kunststoff oder einem anderen elastischen Material gebildet sein.

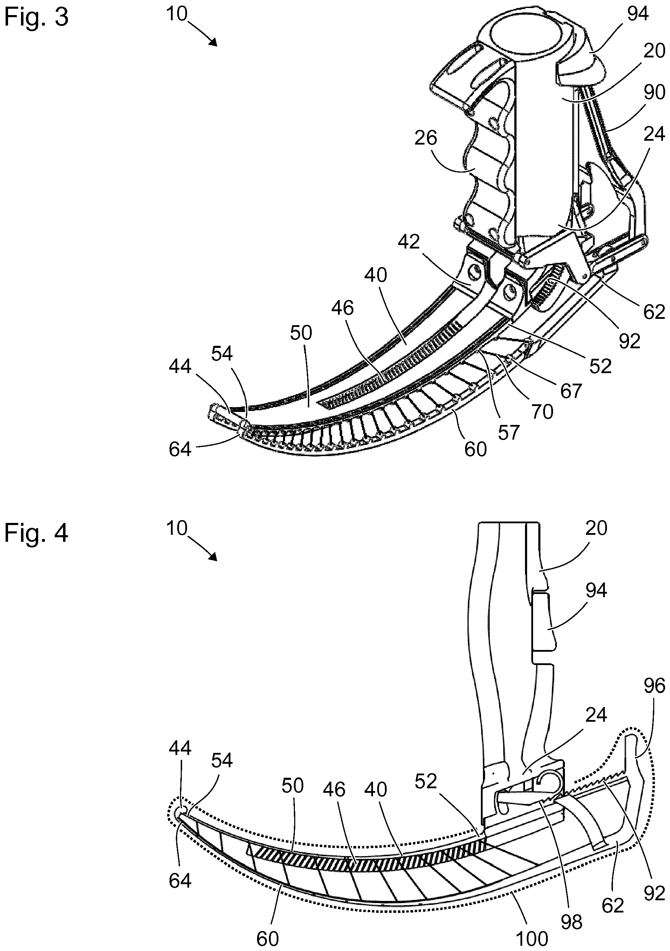

Figur 3 zeigt eine schematische axonometrische Darstellung eines weiteren adaptiven Intubationslaryngoskops 10, das dem anhand der Figur 1 dargestellten Intubationslaryngoskop in einigen Merkmalen, Eigenschaften und Funktionen ähnelt. Nachfolgend sind Merkmale, Eigenschaften und Funktionen des Intubationslaryngoskops 10 beschrieben, in denen dieses sich von dem anhand der Figur 1 dargestellten unterscheidet.

An den Rändern des ersten biegbaren Holms 50 und an den zweiten biegbaren Holmen 60 sind jeweils Ösen 57, 67 angeordnet. Ein Draht 70 aus einer pseudoelastischen Nickel-Titan-Legierung oder ein anderes dünnes und biegbares Bauteil ist alternierend durch zwei benachbarte Ösen 57 am ersten biegbaren Holm 50 und durch zwei benachbarte Ösen 67 an einem der zweiten biegbaren Holme 60 geführt, so dass der Draht eine polygonal mäandernde Gestalt aufweist. Anders ausgedrückt weist der Draht zahlreiche untereinander im Wesentlichen parallele Abschnitte auf, die jeweils den ersten biegbaren Holm 50 mit einem der zweiten biegbaren Holme 60 verbinden. Der Draht 70 überträgt Zugkräfte zwischen den biegbaren Holmen 50, 60 und bewirkt, dass deren Abstände vorbestimmte Maximalwerte nicht überschreiten können. Der Draht kann in den Ösen 57, 67 jeweils fixiert oder in seiner Längsrichtung verschiebbar sein.

Am ersten biegbaren Holm 50 ist ein Kanal 46 zum Führen eines flexiblen Endoskops oder eines anderen medizinischen Instruments vorgesehen. Der Kanal 46 ist durch eine Vielzahl benachbarter bogenförmiger bzw. Omega- oder U-förmiger Bereiche am ersten biegbaren Holm 50 gebildet. Dadurch bewirkt der Kanal 46 keine Versteifung des ersten biegbaren Holms 50.

Alternativ oder zusätzlich kann eine Lichtquelle, beispielsweise eine Leuchtdiode, oder ein Lichtleitkabel zum Übertragen von Beleuchtungslicht vorgesehen sein, wobei die Lichtquelle bzw. die Lichtaustrittsfläche insbesondere an oder nahe dem distalen Ende des adaptiven Spatels angeordnet ist. Alternativ oder zusätzlich kann eine Kamera oder ein Bildsensor mit Objektiv oder ein Endoskop in das adaptive Intubationslaryngoskop 10 integriert sein, wobei die Kamera bzw. der Bildsensor mit Objektiv bzw. das distale Ende des Endoskops an oder nahe dem distalen Ende des adaptiven Spatels angeordnet ist.

Das Griffbauteil 20 weist an einer Seite, an der bei bestimmungsgemäßer Verwendung die Finger einer das Griffbauteil 20 haltenden Hand anliegen, eine ausgeprägte Profilierung 26 auf. An einer gegenüberliegenden Seite des Griffbauteils 20, an der bei bestimmungsgemäßer Verwendung der Handballen anliegt, ist ein Hebel 90 angeordnet, dessen freies Ende mit den proximalen Enden 62 der zweiten biegbaren Holme 60 mechanisch gekoppelt ist. Der Hebel 90 ist mit den proximalen Enden 62 der zweiten biegbaren Holme 60 derart gekoppelt, dass ein Druck auf den Hebel 90 zum Griffbauteil 20 hin eine Verschiebung der zweiten biegbaren Holme 60 nach distal und eine Verstärkung der Krümmung des adaptiven Spatels 40 bewirkt.

Zur Fixierung einer eingestellten Krümmung des adaptiven Spatels 40 ist eine Rasteinrichtung 92 vorgesehen. Die Rasteinrichtung 92 bildet eine Einrichtung zum alternativen Arretieren der proximalen Enden 62 der zweiten biegbaren Holme 60 in einer von mehreren alternativen Positionen relativ zu dem proximalen Ende 52 des ersten biegbaren Holms 50. Ferner ist am Griffbauteil 20 ein Schieber 94 vorgesehen, der mit der Rasteinrichtung 92 derart mechanisch gekoppelt ist, dass eine manuelle Verschiebung des Schiebers 94 in eine vorbestimmte Richtung ein Dearretieren bzw. ein Lösen der Fixierung der eingestellten Krümmung bewirkt.

Figur 4 zeigt eine schematische Darstellung eines weiteren adaptiven Intubationslaryngoskops 10, das den anhand der Figuren 1 bis 3 dargestellten Intubationslaryngoskopen, insbesondere dem anhand der Figur 3 dargestellten Intubationslaryngoskop, in einigen Merkmalen, Eigenschaften und Funktionen ähnelt. Nachfolgend sind insbesondere Merkmale, Eigenschaften und Funktionen des Intubationslaryngoskops 10 beschrieben, in denen dieses sich von dem anhand der Figur 3 dargestellten unterscheidet.

Bei dem in Figur 4 gezeigten adaptiven Intubationslaryngoskop 10 sind der erste biegbare Holm 50 und die zweiten biegbaren Holme 60 durch Streben 74 miteinander verbunden. Die Streben 74 können den anhand der Figur 1 beschriebenen ähneln. Alternativ können die Streben 74 als kurze Drahtstücke oder Garn- bzw. Fadenstücke aus Metall, Kunststoff oder einem anderen elastischen Material ausgebildet sein, deren Enden mit den biegbaren Holmen 50, 60 form-, kraft- oder stoffschlüssig verbunden sind. Alternativ können die biegbaren Holme 50, 60 durch einen Draht aus einer pseudoelastischen Nickel-Titan-Legierung oder ein anderes dünnes und biegbares Bauteil gebildet sein, das alternierend am ersten biegbaren Holm 50 und am zweiten biegbaren Holme 60 befestigt ist, beispielsweise ähnlich wie bei dem anhand der Figur 3 dargestellten Beispiel.

Bei dem in Figur 4 gezeigten adaptiven Intubationslaryngoskop 10 sind die proximalen Enden 62 der zweiten biegbaren Holme 60 mit einer manuell verschiebbaren Druckplatte 96 starr gekoppelt, insbesondere einstückig ausgebildet. Durch eine manuelle Verschiebung der Druckplatte 96 nach distal und damit zum Griffbauteil 20 hin können die zweiten biegbaren Holme 60 nach distal verschoben werden. Dies hat aufgrund der Streben 74 zwischen dem ersten biegbaren Holm 50 und den zweiten biegbaren Holmen 60 eine Verstärkung der Krümmung des adaptiven Spatels 40 zur Folge.

Eine Rasteinrichtung 92 ist starr mit der Druckplatte 96 verbunden. Am Verbindungsbereich 24 des Griffbauteils 20 und des Spatels 40 sind bewegbare Rasthaken 98 vorgesehen.

Die Rasteinrichtung 92 kann in mehreren alternativen vorbestimmten Positionen an den Rasthaken 98 einhaken, um mehrere entsprechende Krümmungen des adaptiven Spatels 40 zu fixieren. Ein Schieber 94 am Griffbauteil 20 ist derart mit den bewegbaren Rasthaken 98 gekoppelt, dass eine manuelle Verschiebung des Schiebers 94 in eine vorbestimmte Richtung ein Dearretieren bzw. ein Lösen der Fixierung der eingestellten Krümmung bewirkt.

Der adaptive Spatel 40 ist optional von einem elastischen Mantelbauteil 100 umgeben, dessen Konturen in Figur 4 durch eine gestrichelte Linie angedeutet sind. Das elastische Mantelbauteil 100 ist beispielsweise aus einer elastischen Kunststofffolie oder aus einem insbesondere mit einer fluiddichten Beschichtung versehenen elastischen Gewebe gebildet. Das elastische Mantelbauteil 100 schützt den adaptiven Spatel 40 vor Verschmutzung und kann beispielsweise nach jeder Verwendung entsorgt und ersetzt werden. Alternativ und abweichend von der Darstellung in Figur 4 kann das Mantelbauteil 100 innenliegend angeordnet sein.

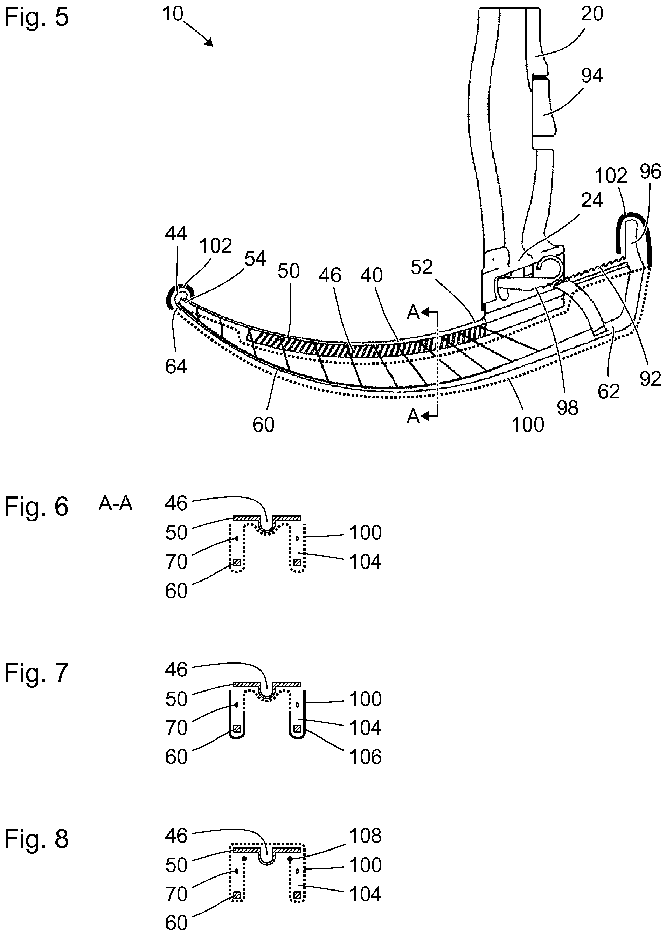

Figur 5 zeigt eine schematische Darstellung eines weiteren adaptiven Intubationslaryngoskops 10, das den anhand der Figuren 1 bis 4 dargestellten Intubationslaryngoskopen, insbesondere dem anhand der Figur 4 dargestellten Intubationslaryngoskop, in einigen Merkmalen, Eigenschaften und Funktionen ähnelt. Nachfolgend sind insbesondere Merkmale, Eigenschaften und Funktionen des Intubationslaryngoskops 10 beschrieben, in denen dieses sich von dem anhand der Figur 4 dargestellten unterscheidet.

Das adaptive Intubationslaryngoskop 10 weist ein elastisches Mantelbauteil 100, das dem anhand der Figur 4 dargestellten elastischen Mantelbauteil ähnelt, auf. Das elastische Mantelbauteil 100 weist hakenartige Einrichtungen 102 (insbesondere Enden) auf, die am distalen Ende 44 des adaptiven Spatels 40 und an der Druckplatte 96 des adaptiven Intubationslaryngoskops 10 eingehängt sind.

Figur 6 zeigt eine schematische Darstellung eines Schnitts durch das adaptive Intubationslaryngoskops 10 aus Figur 5 entlang der in Figur 5 angedeuteten Ebene A-A orthogonal zur Zeichenebene der Figur 5. In Figur 6 ist erkennbar, dass das elastische Mantelbauteil 100 zwischen den beiden zweiten biegbaren Holmen 60 eine rinnenförmige oder U-förmige Gestalt einnimmt und bis zum ersten biegbaren Holm 50 reicht. Das elastische Mantelbauteil 100 bildet zwei Taschen oder Nuten 104, in denen die zweiten biegbaren Holme 60 angeordnet sind.

Das elastische Mantelbauteil 100 ist zwischen den hakenartigen Einrichtungen 102 an seinen Ende so gespannt, dass das elastische Mantelbauteil aufgrund der Krümmung des adaptiven Spatels 40 zuverlässig in der in den Figuren 5 und 6 angedeuteten Konfiguration gehalten ist. Dazu können unterschiedliche Elastizitäten des elastischen Mantelbauteils im Bereich seiner Ränder, in den Bereichen der zweiten biegbaren Holme 60 und in seinem mittleren, zum ersten biegbaren Holm 50 benachbarten Bereich beitragen.

Figur 7 zeigt eine schematische Darstellung eines Schnitts durch ein weiteres adaptives Intubationslaryngoskop 10, das in einigen Merkmalen, Eigenschaften und Funktionen den anhand der Figuren 1 bis 6 dargestellten adaptiven Intubationslaryngoskopen ähnelt. Nachfolgend sind insbesondere Merkmale, Eigenschaften und Funktionen des Intubationslaryngoskops 10 beschrieben, in denen dieses sich von den anhand der Figuren 4 bis 6 dargestellten unterscheidet.

Das adaptive Intubationslaryngoskop 10 weist ein elastisches Mantelbauteil 100, das den anhand der Figuren 4 bis 6 dargestellten elastischen Mantelbauteilen ähnelt, auf. Das elastische Mantelbauteil umfasst zwei versteifte Randbereiche 106, die die Taschen bzw. Nuten 104 für die zweiten biegbaren Holme 60 bilden. Die versteiften Randbereiche 106 sind insbesondere derart versteift, dass Ihre U-förmigen und die Taschen bzw. Nuten 104 bildenden Querschnitte auch bei den größten Kräften, die bei bestimmungsgemäßer Verwendung des adaptiven Intubationslaryngoskops auftreten können, zuverlässig erhalten bleiben.

Figur 8 zeigt eine schematische Darstellung eines Schnitts durch ein weiteres adaptives Intubationslaryngoskop 10, das in einigen Merkmalen, Eigenschaften und Funktionen den anhand der Figuren 1 bis 7 dargestellten adaptiven Intubationslaryngoskopen ähnelt. Nachfolgend sind insbesondere Merkmale, Eigenschaften und Funktionen des Intubationslaryngoskops 10 beschrieben, in denen dieses sich von den anhand der Figuren 4 bis 7 dargestellten unterscheidet.

Das adaptive Intubationslaryngoskop 10 weist ein elastisches Mantelbauteil 100, das den anhand der Figuren 4 bis 7 dargestellten elastischen Mantelbauteilen ähnelt, auf. Das elastische Mantelbauteil umfasst zwei Zugeinrichtungen 108, beispielsweise Drähte, Litzen oder Garne aus Gummi oder einem anderen zugelastischen Material. Die Zugeinrichtungen 108 verbinden die hakenartigen Einrichtungen 102 des elastischen Mantelbauteils 100. Die Zugeinrichtungen 108 können eine Zugelastizität, die geringer ist als die benachbarter Bereiche, aufweisen. Die Zugeinrichtungen 108 halten die Ränder des elastischen Mantelbauteils 100 nahe dem ersten biegbaren Holm 50 und damit zwischen den zweiten biegbaren Holmen 60. Dadurch verhindern oder erschweren die Zugeinrichtungen 108 ein seitliches Abrutschen des elastischen Mantelbauteils 100 von dem adaptiven Spatel 40.

Einige Merkmale und Eigenschaften des anhand der Figur 3 dargestellten adaptiven Intubationslaryngoskops können mit Merkmalen und Eigenschaften des anhand der Figuren 1 und 2 dargestellten adaptiven Intubationslaryngoskops kombiniert werden. Einige Merkmale und Eigenschaften der anhand der Figuren 4 bis 6 dargestellten adaptiven Intubationslaryngoskope können mit Merkmalen und Eigenschaften der anhand der Figuren 1 bis 3 dargestellten adaptiven Intubationslaryngoskope kombiniert werden. Die anhand der Figuren 1 bis 3 dargestellten Intubationslaryngoskope können mit elastischen Mantelbauteilen mit den anhand der Figuren 4 bis 8 dargestellten Merkmalen, Eigenschaften und Funktionen versehen sein.

Bei allen anhand der Figuren 1 bis 4 dargestellten adaptiven Intubationslaryngoskopen können anstelle zweier zweiter biegbarer Holme 60 nur ein zweiter biegbarer Holm vorgesehen sein. Dieser eine zweite biegbare Holm 60 kann plattenförmig bzw. mit einem im Wesentlichen breiten und dünnen Querschnitt ausgebildet sein, ähnlich wie es für den ersten biegbaren Holm 50 beschrieben ist. Alternativ können bei allen anhand der Figuren 1 bis 4 dargestellten adaptiven Intubationslaryngoskopen drei oder mehr zweite biegbare Holme vorgesehen sein.

### Bezugszeichen

- 10: Intubationslaryngoskop
- 20: Griffbauteil des Intubationslaryngoskops 10
- 24: Verbindungsbereich des Griffbauteils 20 und des Spatels 40
- 26: Profilierung am Griffbauteil 20
- 40: adaptiver Spatel des Intubationslaryngoskops 10 oder für das Intubationslaryngoskop 10
- 42: proximales Ende des adaptiven Spatels 40
- 44: distales Ende des adaptiven Spatels 40
- 46: Kanal für ein medizinisches Instrument am adaptiven Spatel 40
- 50: erster biegbarer Holm des adaptiven Spatels 40
- 52: proximales Ende des ersten biegbaren Holms 50
- 54: distales Ende des ersten biegbaren Holms 50
- 55: T-förmige Einrichtung am ersten biegbaren Holm 50 zur formschlüssigen Verbindung mit dem zweiten biegbaren Holm 60
- 56: konvexer, insb. balkenförmiger Bereich am T-förmigen Abschnitt 55 am ersten biegbaren Holm 50
- 57: Öse am ersten biegbaren Holm 50
- 60: zweiter biegbarer Holm des adaptiven Spatels 40
- 62: proximales Ende des zweiten biegbaren Holms 60
- 64: distales Ende des zweiten biegbaren Holms 60
- 65: T-förmige Einrichtung am zweiten biegbaren Holm 60 zur formschlüssigen Verbindung mit dem ersten biegbaren Holm 50
- 66: konkaver, insb. schlitzartiger Bereich hinter der T-förmigen Einrichtung 65 am zweiten biegbaren Holm 60
- 67: Öse am zweiten biegbaren Holm 60
- 68: bogenförmiger Abschnitt am proximalen Ende des zweiten biegbaren Holms 60
- 70: Draht oder anderes dünnes und biegbares Bauteil
- 74: Strebe zwischen den biegbaren Holmen 50, 60
- 75: mit dem ersten biegbaren Holm 50 verbundenes erstes Ende de Strebe 74
- 76: mit dem zweiten biegbaren Holm 60 verbundenes zweites Ende der Strebe 74
- 80: Speicheneinrichtung zur unmittelbaren oder mittelbaren Verbindung des bogenförmigen Abschnitts 68 am proximalen Ende 62 des zweiten biegbaren Holms 60 mit dem proximalen Ende 52 des ersten biegbaren Holms 50
- 85: unmittelbar oder mittelbar mit dem proximalen Ende 52 des ersten biegbaren Holms 50 mechanisch verbundenes erstes Ende der Speicheneinrichtung 80
- 86: unmittelbar oder mittelbar mit dem bogenförmigen Abschnitt 68 am proximalen Ende 62 des zweiten biegbaren Holms 60 mechanisch verbundenes zweites Ende der Speicheneinrichtung 80
- 90: Schieber oder Hebel zum manuellen Bewegen des proximalen Endes 62 des zwei-ten biegbaren Holms (60) relativ zum proximalen Ende 52 des ersten biegbaren Holms 50
- 92: Rasteinrichtung zum alternativen Arretieren des proximalen Endes 62 des zweiten biegbaren Holms (60) in einer von mehreren Positionen relativ zum proximalen Ende 52 des ersten biegbaren Holms 50
- 94: Schieber zur Dearretierung
- 96: Druckplatte
- 98: bewegbare Rasthaken
- 100: elastisches Mantelbauteil zum Schutz des adaptiven Spatels 40 vor Verschmutzung und anderen Umwelteinflüssen
- 102: hakenartige Einrichtung des elastischen Mantelbauteils 100
- 104: Tasche oder Nut am Rand des elastischen Mantelbauteils 100
- 106: versteifter Randbereich des elastischen Mantelbauteils 100
- 108: Zugeinrichtung am Rand des elastischen Mantelbauteils 100

## Patentansprüche

1. **Adaptiver Spatel** (40) für ein Laryngoskop (10), mit:
einem **proximalen Ende** (42), das mit einem Griffbauteil (20) mechanisch verbindbar oder verbunden ist, um ein adaptives Laryngoskop (10) zu bilden;
einem **ersten biegbaren Holm** (50), der sich vom proximalen Ende (42) des adaptiven Spatels (40) bis zu dessen distalem Ende (44) erstreckt;
einem **zweiten biegbaren Holm** (60), der sich vom proximalen Ende (42) des adaptiven Spatels (40) bis zu dessen distalem Ende (44) erstreckt,
wobei am distalen Ende (44) des adaptiven Spatels (40) die biegbaren Holme (50, 60) miteinander mechanisch starr oder gelenkig verbunden sind,
wobei die biegbaren Holme (50, 60) proximal des distalen Endes (44) des adaptiven Spatels (40) derart miteinander mechanisch verbunden sind, dass sie relativ zu einander im Wesentlichen in ihren Längsrichtungen bewegbar sind,
**gekennzeichnet durch**
eine Strebe (74), die proximal des distalen Endes (44) des adaptiven Spatels (40) den ersten biegbaren Holm (50) und den zweiten biegbaren Holm (60) miteinander mechanisch verbindet,
wobei zumindest entweder die Enden (75, 76) der Strebe (74) durch Festkörpergelenke oder andere Gelenke mit den biegbaren Holmen (50, 60) verbunden sind oder die gesamte Strebe elastisch ausgebildet ist.

2. Adaptiver Spatel (40) nach dem vorangehenden Anspruch, ferner mit:
einem **dünnen und biegbaren Verbindungsbauteil** (70), das **abwechselnd** mit dem ersten biegbaren Holm (50) und mit dem zweiten biegbaren Holm (60) verbunden ist.

3. Adaptiver Spatel (40) nach dem vorangehenden Anspruch, bei dem das dünne und biegbare Verbindungsbauteil (70) derart abwechselnd mit dem ersten biegbaren Holm (50) und mit dem zweiten biegbaren Holm (60) verbunden ist, dass das dünne und biegbare Verbindungsbauteil (70) eine mäandernde Gestalt aufweist.

4. Adaptiver Spatel (40) nach dem vorangehenden Anspruch, ferner mit:
**Ösen** (57, 67) oder anderen Führungseinrichtungen an den biegbaren Holmen (50, 60),
wobei das dünne und biegbare Verbindungsbauteil (70) **abwechselnd** durch Ösen (57) oder andere Führungseinrichtungen am ersten biegbaren Holm (50) und durch Ösen (67) oder andere Führungseinrichtungen am zweiten biegbaren Holm (60) geführt ist.

5. Adaptiver Spatel (40) nach dem vorangehenden Anspruch, bei dem das dünne und biegbare Verbindungsbauteil (70) in den Ösen (57, 67) oder anderen Führungseinrichtungen jeweils in seiner Längsrichtung **verschiebbar** gelagert ist.

6. Adaptiver Spatel (40) nach einem der Ansprüche 2 bis 5, bei dem das dünne und biegbare Verbindungsbauteil (70) einen Draht aus einer **pseudoelastischen Nickel-Titan-Legierung** oder aus einem anderen pseudoelastischen Material oder einen Draht aus einem anderen Material umfasst.

7. Adaptiver Spatel (40) nach einem der vorangehenden Ansprüche, ferner mit:
einem **bogenförmigen Abschnitt** (68) am proximalen Ende (62) des zweiten biegbaren Holms (60).

8. Adaptiver Spatel (40) nach dem vorangehenden Anspruch, ferner mit:
**Speicheneinrichtungen** (80), deren erste Enden (85) mit dem proximalen Ende des ersten biegbaren Holms (50) mechanisch verbunden sind, und deren zweite Enden (86) mit dem bogenförmigen Abschnitt (68) des zweiten biegbaren Holms (60) mechanisch verbunden sind.

9. Adaptiver Spatel (40) nach einem der vorangehenden Ansprüche, ferner mit:
einer **formschlüssigen Verbindung** (56, 66) der beiden biegbaren Holme (50, 60) proximal des distalen Endes (44) des adaptiven Spatels (40), die eine Bewegung der beiden biegbaren Holme (50, 60) relativ zu einander in einer ersten vorbestimmten Richtung ermöglicht und eine Bewegung der beiden biegbaren Holme (50, 60) relativ zu einander in einer zweiten vorbestimmten Richtung, die zu der ersten vorbestimmten Richtung orthogonal ist, formschlüssig unterbindet.

10. Adaptiver Spatel (40) nach dem vorangehenden Anspruch, bei dem
die formschlüssige Verbindung durch eine erste Einrichtung (55) am ersten biegbaren Holm (50) und eine zweite Einrichtung (65) am zweiten biegbaren Holm (60) gebildet wird,
ein **konvexer Bereich** (56) an der ersten Einrichtung (55) am ersten biegbaren Holm (50) in einen **konkaven Bereich** (66) an der zweiten Einrichtung (65) am zweiten biegbaren Holm (60) eingreift und innerhalb dessen in der ersten vorbestimmten Richtung verschiebbar ist.

11. Adaptiver Spatel (40) nach einem der vorangehenden Ansprüche, wobei der adaptive Spatel (40) **mehrere zweite biegbare Holme** (50), die parallel oder im Wesentlichen parallel zu einander angeordnet und von einander beabstandet sind, umfasst.

12. Adaptiver Spatel (40) nach einem der vorangehenden Ansprüche, ferner mit:
einem Kanal (46), in den zumindest entweder ein Endoskop oder eine Lichtquelle oder ein anderes medizinisches Instrument eingeführt werden kann.

13. **Adaptives Laryngoskop** (10) mit:
einem adaptiver **Spatel** (40) nach einem der vorangehenden Ansprüche;
einem **Griffbauteil** (20), das mit dem proximalen Ende (42) des adaptiver Spatels (40) mechanisch verbindbar oder verbunden ist.

14. Adaptives Laryngoskop (10) nach dem vorangehenden Anspruch, ferner mit:
einer Einrichtung (92) zum alternativen Arretieren des proximalen Endes (52, 62) von einem der beiden biegbaren Holme (50, 60) in einer von mehreren alternativen Positionen relativ zum proximalen Ende (62, 52) des anderen der beiden biegbaren Holme (50, 60).

## Claims

1. **Adaptive blade** (40) for a laryngoscope (10), comprising:
a **proximal end** (42), which is mechanically connectable or connected to a handle (20) in order to form an adaptive laryngoscope (10);
a **first flexible bar** (50), which extends from the proximal end (42) of the adaptive blade (40) to the distal end (44) thereof;
a **second flexible bar** (60), which extends from the proximal end (42) of the adaptive blade (40) to the distal end (44) thereof,
wherein, at the distal end (44) of the adaptive blade (40), the flexible bars (50, 60) are connected to each other mechanically rigidly or in an articulated manner,
wherein, in the proximal direction from the distal end (44) of the adaptive blade (40), the flexible bars (50, 60) are mechanically connected to each other in such a way that they are movable relative to each other substantially in their longitudinal directions
**characterised in that**
a strut (74) which, in a proximal direction from the distal end (44) of the adaptive blade (40), mechanically connects the first flexible bar (50) and the second flexible bar (60) to each other;
wherein at least one of the entire strut (74) is made elastic or the ends (75, 76) of the strut (74) are connected to the flexible bars (50, 60) by flexure bearings or by other hinges.

2. Adaptive blade (40) according to the preceding claim, further comprising:
a **thin and flexible connecting component** (70) which is alternately connected to the first flexible bar (50) and to the second flexible bar (60).

3. Adaptive blade (40) according to the preceding claim, wherein the thin and flexible connecting component (70) is alternately connected to the first flexible bar (50) and to the second flexible bar (60) in such a way that the thin and flexible component (70) provides a meandering shape.

4. Adaptive blade (40) according to the preceding claim, further comprising:
eyelets (57, 67) or other guiding mechanisms at the flexible bars (50, 60),
wherein the thin and flexible connecting component (70) is **alternately** guided through eyelets (57) or other guiding mechanisms at the first flexible bar (50) and through eyelets (67) or other guiding mechanisms at the second flexible bar (60).

5. Adaptive blade (40) according to the preceding claim, wherein the thin and flexible connecting component (70) is guided **displaceably,** in each case in its longitudinal direction, in the eyelets (57, 67) or other guiding mechanisms.

6. Adaptive blade (40) according to one of claims 2 to 5, wherein the thin and flexible connecting component (70) comprises a wire made of a **pseudo-elastic nickel-titanium alloy** or of another pseudoelastic material, or a wire made of another material.

7. Adaptive blade (40) according to one of the preceding claims, further comprising:
an **arc-shaped portion** (68) at the proximal end (62) of the second flexible bar (60).

8. Adaptive blade (40) according to the preceding claim, further comprising:
**spoke mechanisms** (80), of which first ends (85) are mechanically connected to the proximal end of the first flexible bar (50), and of which second ends (86) are mechanically connected to the arc-shaped portion (68) of the second flexible bar (60).

9. Adaptive blade (40) according to one of the preceding claims, further comprising:
a **form-fit connection** (56, 66) of the two flexible bars (50, 60) proximally with respect to the distal end (44) of the adaptive blade (40), which form-fit connection (56, 66) facilitates a movement of the two flexible bars (50, 60) relative to each other in a first predetermined direction and, by form-fit engagement, suppresses a movement of the two flexible bars (50, 60) relative to each other in a second predetermined direction, which is orthogonal to the first predetermined direction.

10. Adaptive blade (40) according to the preceding claim, wherein
the form-fit connection is produced by a first mechanism (55) at the first flexible bar (50) and a second mechanism (65) at the second flexible bar (60),
a convex area (56) at the first mechanism (55) at the first flexible bar (50) engages in a **concave area** (66) at the second mechanism (65) at the second flexible bar (60) and is displaceable within the latter in the first predetermined direction.

11. Adaptive blade (40) according to one of the preceding claims, wherein the adaptive blade (40) comprises a **plurality of second flexible bars** (60) arranged parallel or essentially parallel to each other and spaced apart from each other.

12. Adaptive blade (40) according to one of the preceding claims, further comprising:
a channel (46) into which at least one of an endoscope, a light source and another medical instrument can be inserted.

13. **Adaptive laryngoscope** (10) comprising:
an **adaptive blade** (40) according to one of the preceding claims;
a **handle** (20), which is mechanically connectable or connected to the proximal end (42) of the adaptive blade (40).

14. Adaptive laryngoscope (10) according to the preceding claim, further comprising:
a mechanism (92) for alternately locking the proximal end (52, 62) of one of the two flexible bars (50, 60) in one of several alternative positions relative to the proximal end (62, 52) of the other of the two flexible bars (50, 60).

## Revendications

1. **Spatule adaptative** (40) destinée à un laryngoscope (10), ladite spatule comprenant :
une **extrémité proximale** (42) reliée ou pouvant être reliée mécaniquement à un élément de préhension (20) pour former un laryngoscope adaptatif (10) ;
une **première barre flexible** (50) qui s'étend de l'extrémité proximale (42) de la spatule adaptative (40) à l'extrémité distale (44) de celle-ci ;
une **deuxième barre flexible** (60) qui s'étend de l'extrémité proximale (42) de la spatule adaptative (40) à l'extrémité distale (44) de celle-ci,
les barres flexibles (50, 60) étant reliées entre elles de manière mécaniquement rigide ou articulée à l'extrémité distale (44) de la spatule adaptative (40),
les barres flexibles (50, 60) étant reliées mécaniquement entre elles du côté proximal de l'extrémité distale (44) de la spatule adaptative (40) de façon à pouvoir se déplacer sensiblement dans leurs directions longitudinales l'une par rapport à l'autre,
**caractérisée par**
une entretoise (74) qui relie mécaniquement la première barre flexible (50) et la deuxième barre flexible (60) l'une à l'autre du côté proximal de l'extrémité distale (44) de la spatule adaptative (40),
au moins les extrémités (75, 76) de l'entretoise (74) étant reliées aux barres flexibles (50, 60) par des articulations solides ou d'autres articulations, ou toute l'entretoise étant conçue de manière élastique.

2. Spatule adaptative (40) selon la revendication précédente, comprenant en outre :
un **élément de liaison mince et flexible** (70) qui est relié **alternativement** à la première barre flexible (50) et à la deuxième barre flexible (60).

3. Spatule adaptative (40) selon la revendication précédente, dans laquelle l'élément de liaison mince et flexible (70) est relié alternativement à la première barre flexible (50) et à la deuxième barre flexible (60) de sorte que l'élément de liaison mince et flexible (70) a une forme sinueuse.

4. Spatule adaptative (40) selon la revendication précédente, comprenant en outre :
des **œillets** (57, 67) ou d'autres moyens de guidage au niveau des barres flexibles (50, 60),
l'élément de liaison mince et flexible (70) étant guidé **alternativement** par des œillets (57) ou d'autres moyens de guidage au niveau de la première barre flexible (50) et par des œillets (67) ou d'autres moyens de guidage au niveau de la deuxième barre flexible (60).

5. Spatule adaptative (40) selon la revendication précédente, dans laquelle l'élément de liaison mince et flexible (70) est à chaque fois monté de manière **coulissante** dans sa direction longitudinale dans les œillets (57, 67) ou d'autres moyens de guidage.

6. Spatule adaptative (40) selon l'une des revendications 2 à 5, dans laquelle l'élément de liaison mince et flexible (70) comprend un fil en **alliage nickel-titane pseudo-élastique** ou en un autre matériau pseudo-élastique ou un fil en un autre matériau.

7. Spatule adaptative (40) selon l'une des revendications précédentes, comprenant en outre :
une **portion incurvée** (68) à l'extrémité proximale (62) de la deuxième barre flexible (60).

8. Spatule adaptative (40) selon la revendication précédente, comprenant en outre :
des **moyens formant rayons** (80) dont les premières extrémités (85) sont reliées mécaniquement à l'extrémité proximale de la première barre flexible (50) et dont les deuxièmes extrémités (86) sont reliées mécaniquement à la portion incurvée (68) de la deuxième barre flexible (60).

9. Spatule adaptative (40) selon l'une des revendications précédentes, comprenant en outre :
une liaison par **complémentarité de formes** (56, 66) des deux barres flexibles (50, 60) du côté proximal de l'extrémité distale (44) de la spatule adaptative (40), laquelle liaison permet un mouvement des deux barres flexibles (50, 60) l'une par rapport à l'autre dans une première direction prédéterminée et empêche par complémentarité de formes un mouvement des deux barres flexibles (50, 60) l'une par rapport à l'autre dans une deuxième direction prédéterminée qui est orthogonale à la première direction prédéterminée.

10. Spatule adaptative (40) selon la revendication précédente, dans laquelle
la liaison par complémentarité de formes est formée par un premier moyen (55) de la première barre flexible (50) et un deuxième moyen (65) de la deuxième barre flexible (60),
une zone convexe (56) au niveau du premier moyen (55) de la première barre flexible (50) s'engage dans une **zone concave** (66) au niveau du deuxième moyen (65) de la deuxième barre flexible (60) et peut coulisser à l'intérieur de celle-ci dans la première direction prédéterminée.

11. Spatule adaptative (40) selon l'une des revendications précédentes, la spatule adaptative (40) comprenant une **pluralité de deuxièmes barres flexibles** (60) qui sont disposées parallèlement ou sensiblement parallèlement les unes aux autres et espacées les unes des autres.

12. Spatule adaptative (40) selon l'une des revendications précédentes, comprenant en outre :
un canal (46) dans lequel au moins un endoscope ou une source lumineuse ou un autre instrument médical peut être inséré.

13. **Laryngoscope adaptatif** (10) comprenant :
une **spatule** adaptative (40) selon l'une des revendications précédentes ;
un **élément de préhension** (20) qui est relié ou peut être relié mécaniquement à l'extrémité proximale (42) de la spatule adaptative (40).

14. Laryngoscope adaptatif (10) selon la revendication précédente, comprenant en outre :
un moyen (92) destiné à bloquer alternativement l'extrémité proximale (52, 62) de l'une des deux barres flexibles (50, 60) dans l'une parmi une pluralité de positions alternatives par rapport à l'extrémité proximale (62, 52) de l'autre des deux barres flexibles (50, 60).
